# EUROPÄISCHE PATENTSCHRIFT

(11) **EP 0 579 939 B1**
(45) Veröffentlichungstag und Bekanntmachung des Hinweises auf die Patenterteilung: **03.02.1999**
(21) Anmeldenummer: 93108904.9
(22) Anmeldetag: 03.06.1993
(51) Int. Cl.: C07F 9/553, C07F 9/568, C07F 9/572, C07F 9/576, C07F 9/59, C07F 9/547, A61K 31/675

(54) **Neue Phospholipidderivate**
Phospholipid derivatives
Dérivés de phospholipide

(30) Priorität: 11.07.1992 DE 4222910
(43) Veröffentlichungstag der Anmeldung: 26.01.1994
(73) Patentinhaber: ASTA Medica Aktiengesellschaft, D-01277 Dresden (DE)
(72) Erfinder: Nössner, Gerhard, Dr., W-6050 Offenbach (DE); Kutscher, Bernhard, Dr., W-6457 Maintal 1 (DE); Schumacher, Wolfgang, Dr., W-6070 Langen (DE); Engel, Jürgen, Prof., W-8755 Alzenau (DE); Stekar, Jurij, Dr., W-4800 Bielefeld 13 (DE); Hilgard, Peter, Dr., W-4800 Bielefeld 1 (DE)
(74) Vertreter: Decker, Karl-Ludwig

(56) Entgegenhaltungen:
- EP-A- 0 108 565
- EP-A- 0 257 762
- EP-A- 0 284 395

## Beschreibung

Die Europa-Patentanmeldung 108 565 betrifft Verbindungen der allgemeinen Formel 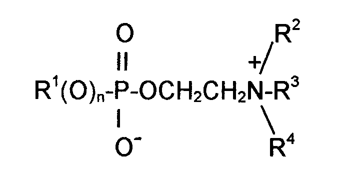 und deren pharmazeutisch verwertbare Salze, worin R₁ ein aliphatischer Kohlenwasserstoffrest mit 8 - 30 C-Atomen ist, die Reste R₂, R₃ und R₄ gleich oder verschieden sind und Wasserstoff oder niedere Alkylreste sind, oder worin die Gruppe NR₂R₃R₄ eine cyclische Ammoniumgruppe bedeutet und n die Werte 0 oder 1 hat. Für diese Verbindungen wird eine Anti-Tumor-Wirkung sowie eine pilzhemmende Wirkung angegeben. Bei einer aus diesem Patent am umfangreichsten geprüften Verbindung, dem Hexadecylphosphocholin, zeigen sich jedoch Nachteile wie schlechte orale Verträglichkeit. Ferner wurde bei Gabe von therapeutisch wirksamen Dosen bei Ratten eine deutliche Gewichtsabnahme festgestellt. Es bestand daher die Aufgabe neue potentielle Arzneimittel bereitzustellen mit einem Wirkungsspektrum als Anti-Tumormittel, Mittel gegen parasitäre Erkrankungen, Autoimmunerkrankungen, Hauterkrankungen sowie Knochenmarksschädigungen, die bei vergleichbar guter Wirksamkeit wie die strukturverwandten Verbindungen eine verbesserte Verträglichkeit aufweisen und deren Anwendung nicht mit einer Verringerung des Körpergewichtes einhergeht. Ferner war es Aufgabe der Erfindung effektive Herstellungs- und Reinigungsverfahren für diese Verbindungen anzugeben.

Die Erfindung betrifft Alkyl- oder Alkenphosphate, deren Cholinrest Teil eines heterozyklischen Rings ist, ein Verfahren zur Herstellung der Verbindungsklasse sowie die Arzneimittel und Verfahren zur Herstellung von Arzneimitteln, die die Verbindungen als Wirkstoffe enthalten.

Die Erfindung betrifft auch Herstellverfahren und Reinigungsverfahren für die neuen Verbindungen.

Die erste Stufe des Verfahrens besteht in der Reaktion von Phosphoroxychlorid mit dem langkettigen Alkohol in halogenierten oder aromatischen Kohlenwasserstoffen, gesättigten cyclischen Ethern oder acyclischen Ethern.

Die erste Stufe des Verfahrens A besteht in der Reaktion von Phosphoroxychlorid mit langkettigem Alkohol in halogenierten Kohlenwasserstoffen, gesättigten cyclischen Ethern, acyclischen Ethern, gesättigten Kohlenwasserstoffen mit 5 bis 10 C-Atomen, flüssigen aromatischen Kohlenwasserstoffen, die auch durch Halogen (insbesondere Chlor) substituiert sein können oder in Gemischen aus den vorstehend erwähnten Lösungsmitteln oder ohne Lösungsmittel, gegebenenfalls in Gegenwart einer hierfür üblichen basischen Substanz.

Als halogenierte Kohlenwasserstoffe kommen beispielsweise Kohlenwasserstoffe alt 1 bis 6 C-Atomen infrage, wobei ein oder mehrere oder alle Wasserstoffatome durch Chloratome ersetzt sind. Beispielsweise können Methylenchlorid, Chloroform, Ethylenchlorid, Chlorbenzol, Dichlorbenzol verwendet werden. Falls es sich um halogensubsituierte aromatische Kohlenwasserstoffe handelt, sind diese vorzugsweise mit einem oder zwei Halogenatomen substituiert.

Als gesättigte cyclische Ether können beispielsweise Ether mit einer Ringröße von 5-6, die aus Kohlenstoffatomen und einem oder 2 Sauerstoffatomen bestehen, verwendet werden.
Beispiele hierfür sind Tetrahydrofuran, Dioxan.

Die acyclischen Ether bestehen aus 2 bis 8 Kohlenstoffatomen und sind flüssig.
Beispielsweise kommen infrage: Diethylether, Diisobutylether, Methyl-tert.-butylether, Diisopropylether.

Als gesättigte Kohlenwasserstoffe kommen unverzweigte und verzweigte Kohlenwasserstoffe, die aus 5 bis 10 Kohlenstoffatomen bestehen und flüssig sind, infrage.

Beispielsweise kommen Pentan, Hexan, Heptan, Cyclohexan infrage.

Als aromatische Kohlenwasserstoffe kommen beispielsweise Benzol und alkylsubstituierte Benzole, wobei die Alkylsubstituenten aus 1 bis 5 Kohlenstoffatomen bestehen, infrage.

Als basische Substanzen sowohl für die Reaktion des Phosphoroxychlorids mit dem langkettigen Alkohol als auch für die anschließende Umsetzung zum Phosphorsäurediester kommen Amine infrage, beispielsweise aliphatische Amine der Formel NR₁R₂R₃, wobei R₁, R₂ und R₃ gleich oder verschieden sind und Wasserstoff oder C₁-C₆-Alkyl bedeuten oder auch aromatische Amine wie Pyridin, Picolin, Chinolin. Bei der Umsetzung zum Phosphorsäurediester kann die hier erforderliche basische Substanz gleichzeitig oder auch vor dem Aminoalkohol beziehungsweise Ammonioalkohol-Salz zugesetzt werden.

Für diese Reaktion ist in jedem Fall ein Lösungsmittel erforderlich; das heißt falls der erste Reaktionsschritt ohne ein besonderes Lösungsmittel erfolgt, muß ein solches jetzt zugegeben werden. Das Molverhältnis von Phosphoroxychlorid zu dem langkettigen Alkohol liegt zum Beispiel zwischen 1,5 : 1 bis 0,8 : 1.

Der Aminoalkohol beziehungsweise das Ammonioalkohol-Salz wird beispielsweise in Bezug auf den langkettigen Alkohol im Überschuß eingesetzt (etwa 1,1 - 1,5 molarer Überschuß).

Falls die Reaktion des Phosphoroxychlorids mit dem langkettigen Alkohol in Gegenwart einer basischen Substanz erfolgt, ist die Menge der basischen Substanz beispielsweise 1 bis 3 Mol bezogen auf 1 Mol POCl₃. Für die anschließende Umsetzung zum Phosphorsäurediester ist die verwendete Menge an basischer Substanz beispielsweise 1 bis 5 Mol bezogen auf 1 Mol.

Die Reaktionstemperatur der Umsetzung von Phosphoroxychlorid mit dem langkettigen Alkohol liegt zwischen -30°C und +30°C, vorzugsweise -15°C und +5°C, insbesondere -10°C und -5°C.

Die Reaktionszeit dieser Umsetzung beträgt beispielsweise 0,5-5 Stunden, vorzugsweise 1-3 Stunden, insbesondere 1,5-2 Stunden. Falls die Reaktion in Gegenwart einer basischen Substanz erfolgt, verläuft sie im allgemeinen schnell (etwa 30 Minuten).

Danach wird der Aminoalkohol beziehungsweise das Ammonioalkohol-Salz portionsweise oder vollständig zugegeben.
Als Salze des Ammonioalkohols kommen Salze mit Mineralsäuren, (wie zum Beispiel Schwefelsäure, Salzsäure) infrage, ferner Salze mit organischen Säuren, wie zum Beispiel Essigsäure, para-Toluolsulfonsäure und ähnliche.
Dieser Reaktionsschritt erfolgt in einem inerten Lösungsmittel. Als Lösungsmittel kommen hier die gleichen infrage, die für die Umsetzung des Phosphoroxychlorids mit dem langkettigen Alkohol verwendet werden, falls diese Umsetzung in einem Lösungsmittel erfolgt.

Anschließend wird die basische Substanz in einem der angegebenen Lösungsmittel gelöst, oder ohne Lösungsmittel zugetropft.
Vorzugsweise werden als Lösungsmittel für die basische Substanz hier verwendet: Halogenierte Kohlenwasserstoffe, gesättigte cyclische Ether, acyclische Ether, gesättigte Kohlenwasserstoffe mit 5 bis 10 Kohlenstoffatomen, flüssige aromatische Kohlenwasserstoffe oder Gemische aus vorstehend erwähnten Lösungsmitteln.

Es handelt sich hier um die gleichen Lösungsmittel, wie sie für die Umsetzung des Phosphoroxychlorids mit dem langkettigen Alkohol verwendet werden können.

Durch die Zugabe der basischen Substanz steigt die Temperatur. Man sorgt dafür, daß die Temperatur in einem Bereich zwischen 0°C bis 40°C, vorzugsweise 10°C bis 30°C, insbesondere auf 15°C bis 20°C gehalten wird.

Die Reaktionsmischung wird dann noch bei 5°C bis 30°C, vorzugsweise 15°C bis 25°C gerührt, (beispielsweise 1 Stunde bis 40 Stunden, vorzugsweise 3 Stunden bis 15 Stunden.)

Die Hydrolyse des Reaktionsansatzes erfolgt durch Zugabe von Wasser, wobei eine Temperatur zwischen 10°C und 30°C, vorzugsweise 15°C und 30°C, insbesondere zwischen 15°C und 20°C eingehalten werden soll.

Die zuvor erwähnten Hydrolyseflüssigkeiten können auch basische Substanzen enthalten. Als solche basischen Substanzen kommen Carbonate und Hydrogencarbonate der Alkali- und Erdalkalimetalle infrage.

Zur Vervollständigung der Hydrolyse wird anschließend noch 0,5 Stunden bis 4 Stunden, vorzugsweise 1 bis 3 Stunden, insbesondere 1,5 bis 2,5 Stunden bei 10°C bis 30°C, vorzugsweise bei 15°C bis 25°C, insbesondere bei 18°C bis 22°C gerührt.

Die Reaktionslösung wird dann mit einem Gemisch aus Wasser und Alkoholen (vorzugsweise aliphatische gesättigte Alkohole mit 1 bis 4 Kohlenstoffatomen), das gegebenenfalls noch eine basische Substanz enthalten kann, gewaschen.
Das Mischungsverhältnis Wasser: Alkohol kann beispielsweise zwischen 5 und 0,5 liegen, vorzugsweise 1-3 (V/V).

Als basische Substanzen für die Waschflüssigkeit kommen zum Beispiel Carbonate und Hydrogencarbonate der Alkali- und Erdalkalimetalle sowie Ammoniak (zum Beispiel wässeriges Ammoniak) infrage. Besonders bevorzugt ist eine 3%ige Natriumcarbonatlösung in Wasser.

Gegebenenfalls kann anschließend eine waschung der Reaktionslösung mit einer sauren Lösung vorgenommen werden.
Vorteilhaft ist die saure Waschung zur Entfernung noch nicht umgesetzter basischer Anteile der Reaktionslösung, insbesondere bei der Verwendung von Methylenchlorid als Lösungsmittel.

Die Waschlösung besteht aus einem Gemisch aus Wasser und Alkoholen. Vorzugsweise kommen Mischungen aus aliphatischen gesättigten Alkoholen mit 1 bis 4 Kohlenstoffatomen infrage, wobei gegebenenfalls noch eine saure Substanz anwesend sein kann. Das Mischungsverhältnis Wasser:Alkohol kann beispielsweise zwischen 5 und 0,5 liegen, vorzugsweise 1-3 (V/V).

Als saure Substanz für die Waschflüssigkeit kommen zum Beispiel Mineralsäuren und organische Säuren infrage, zum Beispiel Salzsäure, Schwefelsäure, Weinsäure oder Zitronensäure. Besonders bevorzugt ist eine 10% Lösung von Salzsäure in Wasser.

Anschließend wird noch einmal mit einem Gemisch aus Wasser und Alkoholen gewaschen. Vorzugsweise kommen Mischungen aus aliphatischen gesättigten Alkoholen mit 1 bis 4 Kohlenstoffatomen infrage, wobei gegebenenfalls noch eine basische Substanz anwesend sein kann.
Das Mischungsverhältnis Wasser: Alkohol kann beispielsweise zwischen 5 und 0,5 liegen, vorzugsweise 1-3.

Die gewaschenen Phasen werden dann vereinigt und in üblicher Weise getrocknet, und dann das Lösungsmittel (vorzugsweise unter vermindertem Druck, zum Beispiel 5 bis 100 mbar) gegebenenfalls nach Zugabe von 150 - 1000 ml, vorzugsweise 300 - 700 ml, insbesondere 450 - 550 ml eines aliphatischen Alkohols, entfernt (bezogen auf 1 Mol Gewichtsteil trockenes Produkt).
Als Alkohole kommen vorzugsweise gesättigte aliphatische Alkohole mit einer Kettenlänge von 1 und 5 Kohlenstoffatomen in Frage. Besonders bevorzugt als Alkohol ist hierbei n-Butanol, Isopropanol. Diese Alkoholbehandlung hat den Zweck der vollständigen Entfernung von Restwasser und Vermeidung von Schaumbildung.

Die weitere Reinigung des Produkts kann beispielsweise erfolgen, indem man das Rohprodukt heiß in Ethanol löst, vom Rückstand abfiltriert und mit einem Mischbettionenaustauscher, wie zum Beispiel Amberlite MB3 in ethanolischer Lösung behandelt. Anstelle eines Mischbettionenaustauschers können auch alle handelsüblichen sauren und basischen Ionenaustauscher gleichzeitig oder nacheinander eingesetzt werden.

Die Lösung wird anschließend aus Ketonen, wie zum Beispiel Aceton oder Methylethylketon umkristallisiert, in einigen Fällen ist digerieren mit den obigen Lösungsmitteln ausreichend.
Es kann zweckmäßig sein, die Produkte durch Säulenchromatographie beziehungsweise Flash-Chromatographie an Kieselgel zu reinigen. Als Laufmittel werden beispielsweise Gemische aus Chloroform, Methylenchlorid, Methanol und 25 % Ammoniaklösung eingesetzt.

Die Verfahrensvariante B besteht in der nachträglichen Alkylierung von Produkten, die nach Verfahren A bei Einsatz von Aminoalkoholen erhältlich sind. Als alkylierende Agentien können beispielsweise p-Tolulolsulfonsäuremethylester oder Dimethylsulfat verwendet werden.
Als Lösungsmittel kommen die vorstehend erwähnten Lösungsmittel infrage.

Als basische Substanzen werden beispielsweise Alkalimetallcarbonate verwendet.
Die Umsetzung erfolgt bei erhöhter Temperatur, beispielsweise bei der Siedetemperatur der Lösungsmittel.

### Beispiele:

- Beispiel 1:: Bezeichnung (IUPAC-Nomenklatur)
4-(((Octadecyloxy)hydroxyphosphenyl)oxy)-1,1-dimethylpiperidiniumhydroxid inneres Salz
Kurzbezeichnung:
Octadecyl-(1,1-dimethylpiperidinio-4-yl)-phosphat
C₂₅H₅₂NO₄P (461.66) * 1/2 H₂O

### Herstellvariante A:

10,3 ml (0,11 mol) Phosphoroxychlorid werden in 100 ml Chloroform vorgelegt und auf 5-10 °C abgekühlt. Innerhalb von 30 min. tropft man unter Rühren 27.0 (0.10 mol) 1-Octadecanol, gelöst in 100 ml Chloroform und 35 ml Pyridin, hinzu. Nach 30 min Nachrühren bei 5-10 °C versetzt man mit 39,1 g (0,13 mol) 4-Hydroxy-1,1-dimethylpiperidiniumtosylat in einer Portion. Nach Zugabe von 40 ml Pyridin und 30 ml DMF rührt man 24 h bei Raumtemperatur. Danach hydrolysiert man mit 15 ml Wasser, rührt 30 min nach und wäscht die organische Phase mit je 200 ml Wasser/Methanol (1:1), 3 % Na₂CO₃/Methanol (1:1) und abschließend mit Wasser/Methanol (1:1). Die organische Phase wird eingeengt, der Rückstand in 300 ml Ethanol heiß gelöst und nach dem Abkühlen filtriert. Das Filtrat wird mit 80 g Ionenaustauscher Amberlite MB3 gerührt, abfiltriert und eingeengt. Der Rückstand wird aus 300 ml Methylethylketon umkristallisiert, abgenutscht und im Vakuum über P₂O₅ getrocknet.
Ausbeute: 4.71 g (10 %)

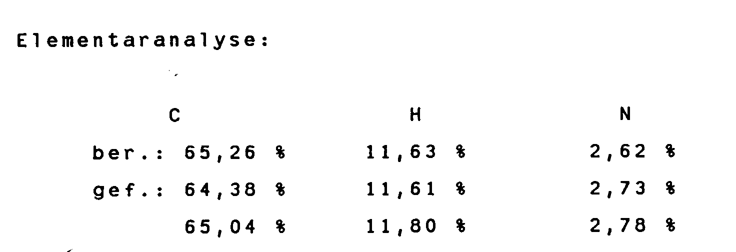

Dünnschichtchromatogramm: (Chloroform/Methanol/1 M Natriumacetat in 25 % Ammoniak 70:40:10)
R_{f} = 0,17
(1-Butanol/Eisessig/Wasser 40:10:10)
R_{f} = 0,12
Schmelzpunkt: 270 - 271 °C (Zersetzung)

### Herstellvariante B:

20.1 ml (0,22 mol) Phosphoroxychlorid werden in 100 ml Methylenchlorid vorgelegt, auf 5-10 °C abgekühlt und unter Rühren innerhalb von 30 min mit einer Lösung von 54,1 g (0,20 mol) Octadecanol in 400 ml Methylenchlorid und 70,5 ml Pyridin versetzt. Nach einer Stunde Nachrühren werden tropfenweise 29,9 g (0,26 mol) 4-Hydroxy-1-methylpiperidin in 80 ml Pyridin zugegeben. Nach 3 h Rühren bei 10 °C hydrolysiert man unter Eiskühlung mit 30 ml Wasser und rührt eine Stunde nach. Die organische Phase wird mit je 200 ml Wasser/Methanol (1:1), 3 prozentiger Salzsäure/Methanol (1:1) und Wasser/Methanol (1:1) gewaschen. Die über Na₂SO₄ getrocknete organische Phase wird bis zur Trübung eingeengt und mit 1 l Methylethylketon versetzt. Das Kristallisat wird aus 1 l Methylethylketon umkristallisiert, abgenutscht und über P₂O₅ im Vakuum getrocknet.
- Ausbeute:: 54.1 g (60 %) Octadecyl-(1-methylpiperidino-4-yl)-phosphat

98,1 g (0,22 mol) Octadecyl-(1-methylpiperidinio-4-yl)-phosphat werden in 500 ml abs. Ethanol suspendiert und zum Sieden erhitzt. Unter Rückfluß gibt man in acht Portionen wechselweise insgesamt 71,8 g (0,39 mol) p-Toluolsulfonsäuremethylester und 26,5 g (0,19 mol) Kaliumcarbonat innerhalb von 2 h zu. Nach beendeter Zugabe erhitzt man noch eine Stunde unter Rückfluß. Nach dem Erkalten filtriert man ab, engt das Filtrat bis zur Hälfte ein und versetzt die Lösung mit 150 g feuchtem Ionenaustauscher Amberlite MB 3. Nach zwei Stunden Rühren wird über Kieselgur/Aktivkohle abgesaugt, das Filtrat eingeengt und unter Aceton kristallisiert. Der Kristallkuchen wird aus Methylethylketon umkristallisiert und im Vakuum über P₂O₅ getrocknet.
Ausbeute: 46,1 g (46 %) Octadecyl-(1,1-dimethylpiperidinio-4-yl)-phosphat

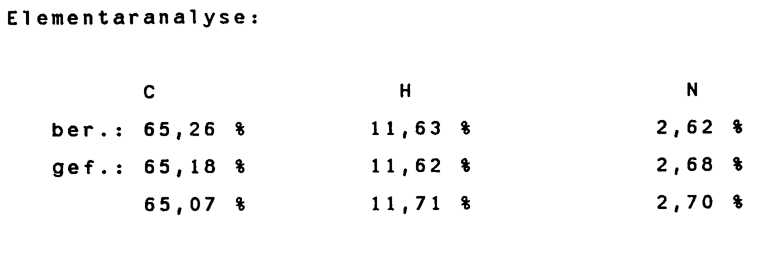

Schmelzpunkt: 271-272 °C (Zersetzung)

### Beispiel 2: Hexadecyl-piperidinio-4-yl-phosphat

### C₂₁H₄₄NO₄P (405,558)

7,1 ml (77 mmol) Phosphoroxychlorid werden in 50 ml trockenem Tetrahydrofuran gelöst und nach dem Abkühlen auf 5-10 °C unter Rühren tropfenweise mit einer Lösung von 17 g (70 mmol) Hexadecanol und 48 ml Triethylamin in 150 ml Tetrahydrofuran versetzt. Nach beendeter Zugabe rührt man 30 min im Eisbad nach und läßt anschließend auf Raumtemperatur kommen. 10.1 g (100 mmol) 4-Piperidinol werden in 100 ml Tetrahydrofuran gelöst, mit 17 ml Triethylamin gemischt und tropfenweise unter Rühren der Reaktionslösung zugegeben, so daß die Temperatur 40 °C nicht übersteigt. Nach beendeter Zugabe wird eine Stunde unter Rückfluß gekocht. Die noch heiße Lösung wird vom Triethylammoniumchlorid abfiltriert und nach dem Abkühlen unter Rühren in eine Eis-2M-Salzsäure-Mischung gegossen. Das beim Abkühlen im Eisschrank anfallende Produkt wird in Methylenchlorid aufgenommen, über MgSO₄ getrocknet und nach dem Einengen an Kieselgel mit Methylenchlorid/Methanol/25 % Ammoniak (70:30:5) chromatographiert. Die das Produkt enthaltenden Fraktionen werden vereinigt und eingeengt. Nach dem Umkristallisieren aus Methanol wird im Vakuum über P₂O₅ getrocknet.
Ausbeute: 10,0 g (35 %)

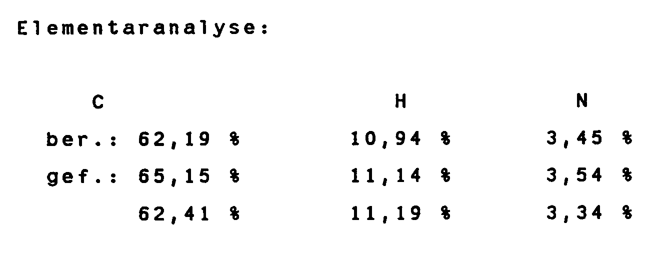

Dünnschichtchromatogramm: (Chloroform/Methanol/25 % Ammoniak 70:20:10)
R_{f} = 0,42
(1-Butanol/Eisessig/Wasser 40:10:10)
R_{f} = 0,33

### Beispiel 3: Hexadecyl-(1,1-dimethylpiperidinio-4-yl)-phosphat

### C₂₃H₄₈NO₄P (433,62) · H₂O

5.7 g (14 mmol) Hexadecyl-piperidinio-4-yl-phosphat werden in 100 ml Methanol gelöst und mit 11,6 g (84 mmol) Kaliumcarbonat gemischt. Unter gutem Rühren werden 4,0 ml (42 mmol) Dimethylsulfat innerhalb von 30 min zugetropft. Die Mischung wird 4 h bei 40 °C nachgerührt, nach dem Abkühlen filtriert und eingeengt. Der Rückstand wird mit Aceton digeriert und nach dem Absaugen in 100 ml 96 % Ethanol gelöst. Man fügt 15 g Ionenaustauscher Amberlite MB 3 zu und rührt 3 h. Nach dem Abfiltrieren wird eingeengt und zweimal aus Methylethylketon umkristallisisert. Trocknen über P₂O₅ im Vakuum.
Ausbeute: 3,70 g (61 %)

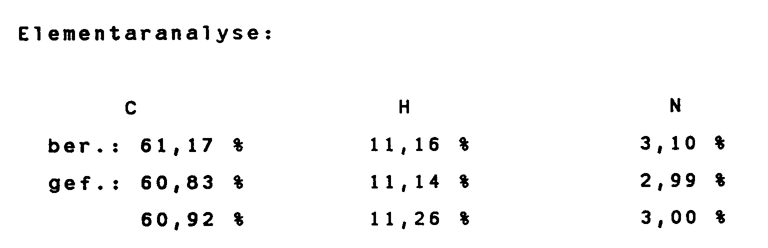

Dünnschichtchromatogramm: (Chloroform/Methanol/25 % Ammoniak 70:20:10)
R_{f} = 0,28
(1-Butanol/Eisessig/Wasser 40:10:10)
R_{f} = 0,13
Schmelzpunkt: 230°C (Zersetzung)

### Beispiel 4: Erucyl-(1,1-dimethylpiperidinio-4-yl)-phosphat

### C₂₉H₅₈NO₄P (515.765) * H₂O

10,3 ml (0,11 mol) Phosphoroxychlorid werden in 50 ml Chloroform vorgelegt und mit einer Lösung von 32,5 g (0,10 mol) Erucylalkohol und 32 ml Pyridin in 100 ml Chloroform tropfenweise bei 5-10 °C versetzt. Nach einer halben Stunde Nachrühren gibt man in einer Portion 39,1 g (0,13 mol) 4-Hydroxy-1,1-dimethylpiperidiniumtosylat zu. Nach Zutropfen von 40 ml Pyridin läßt man auf Raumtemperatur kommen und rührt 3 h. Danach hydrolysiert man mit 15 ml Wasser, rührt eine halbe Stunde nach und wäscht mit je 100 ml Wasser/Methanol (1:1), 3 % Natriumcarbonatlösung/Methanol (1:1), 3 % Citronensäure/ Methanol (1:1) und Wasser/Methanol (1:1). Der nach dem Einengen der organischen Phase erhaltene Rückstand wird mit Aceton digeriert und anschließend in 150 ml 96 % Ethanol gelöst. Diese Lösung wird 3 h mit 20 g Ionenaustauscher Amberlite MB 3 gerührt und über Kieselgur klar abfiltriert. Nach dem Einengen wird am Kieselgel mit Chloroform/ Methanol/25 % Ammoniak 70:40:10 chromatographiert. Die das Produkt enthaltenden Fraktionen werden vereinigt und im Vakuum zur Trockne eingeengt.
Ausbeute: 4.4 g (9 %)

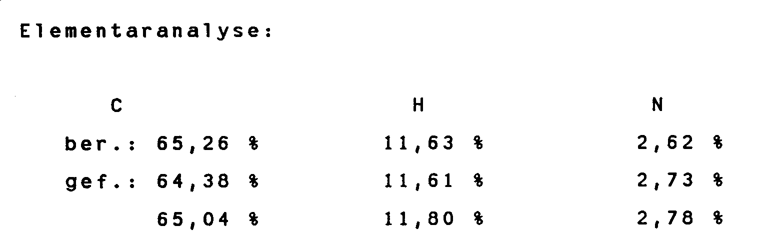

Dünnschichtchromatogramm: (Chloroform/Methanol/1 M Natriumacetat in 25 % Ammoniak 70:20:10)
R_{f} = 0,30

### Beispiel 5: Hexadecyl-(1,1-dimethylpiperidinio-3-yl)-phosphat

### C₂₃H₄₈NO₄P (433.616) * H₂O

10,3 ml (0,11 mol) Phosphoroxychlorid werden in 50 ml Chloroform vorgelegt und auf 0 - 10 °C abgekühlt. 24,2 g (0,10 mol) n-Hexadeca-nol werden in 100 ml Chloroform gelöst, mit 32 ml Pyridin versetzt und innerhalb von einer Stunde unter Eiskühlung zur Phosphoroxychloridlösung getropft. Nach einer halben Stunde Nachrühren gibt man in einer Portion 39,2 g (0,13 mol) 3-Hydroxy-1,1-dimethylpiperidiniumtosylat zu und tropft bei Raumtemperatur innerhalb 15 min 40 ml Pyridin ein. Nach 16 h Rühren bei Raumtemperatur hydrolysiert man mit 15 ml Wasser, rührt eine halbe Stunde und wäscht mit je 100 ml Wasser/Methanol (1:1), 3 % Natriumcarbonatlösung/Methanol (1:1), 3 % Citronensäure/Methanol (1:1) und Wasser/Methanol (1:1). Die organische Phase wird nach dem Trocknen über Natriumsulfat eingeengt. Man löst den Rückstand in 150 ml 96 % Ethanol, filtriert und rührt das Filtrat mit Ionenaustauscher Amberlite MB 3. Nach dem Abfiltrieren von Ionenaustauscher wird eingeengt, der Rückstand mit Aceton zur Kristallisation gebracht und nach dem Absaugen im Vakuum über P₂O₅ getrocknet.
Ausbeute: 13,5 g (31 %)

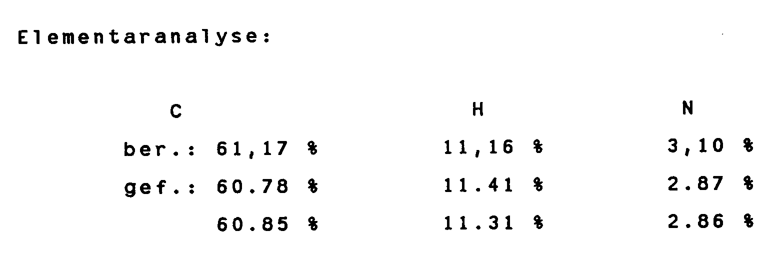

Dünnschichtchromatogramm: (Chloroform/Methanol/1 M Natriumacetat in 25 % Ammoniak 70:40:10)
R_{f} = 0,37

### Beispiel 6: Octadecyl-(1,1-dimethylpiperidinio-3-yl)-phosphat

### C₂₅H₅₂NO₄P (461,670) * 1/2 H₂O

Herstellung analog Beispiel 5 aus 10,3 ml (0,11 mol) Phosphoroxychlorid, 27,0 g (0,10 mol) Octadecanol, 32 + 40 ml Pyridin und 39,2 g (0,13 mol) 3-Hydroxy-1,1-dimethylpiperidiniumtosylat.
Ausbeute: 18,7 g (40 %)

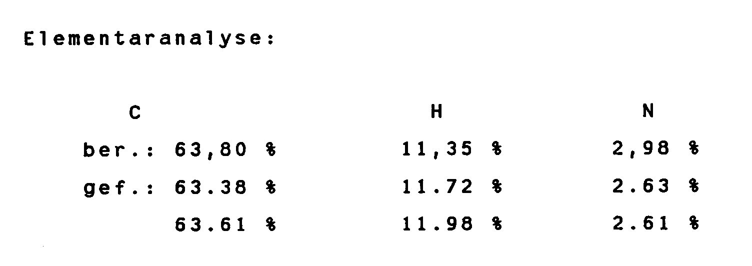

Dünnschichtchromatogramm: (Chloroform/Methanol/1 M Natriumacetat in 25 % Ammoniak 70:40:10)
R_{f} = 0,35

### Beispiel 7: Hexadecyl-(1,1-dimethylpiperidinio-2-yl)methyl-phosphat

### C₂₄H₅₀NO₄P (447.643) * 1/2 H₂O

Herstellung analog Beispiel 5 aus 10,3 ml (0,11 mol) Phosphoroxychlorid, 24,2 g (0,10 mol) Hexadecanol, 32 + 40 ml Pyridin und 41,0 g (0,13 mol) 2-Hydroxymethyl-1,1-dimethylpiperidiniumtosylat.
Ausbeute: 22,9 g (51 %)

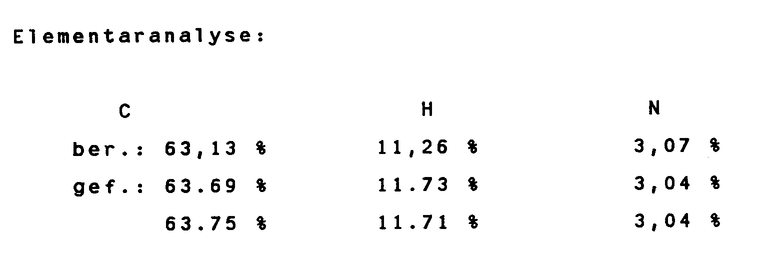

Dünnschichtchromatogramm: (Chloroform/Methanol/1 M Natriumacetat in 25 % Ammoniak 70:40:10)
R_{f} = 0,47

### Beispiel 8: Octadecyl-(1,1-dimethylpiperidinio-2-yl)methyl-phosphat

### C₂₆H₅₄NO₄P (475.697) * 1/2 H₂O

Herstellung analog Beispiel 5 aus 10,3 ml (0,11 mol) Phosphoroxychlorid, 27,0 g (0,10 mol) Octadecanol, 32 + 40 ml Pyridin und 41,0 g (0,13 mol) 2-Hydroxymethyl-1,1-dimethylpiperidiniumtosylat.
Ausbeute: 23,9 g (50 %)

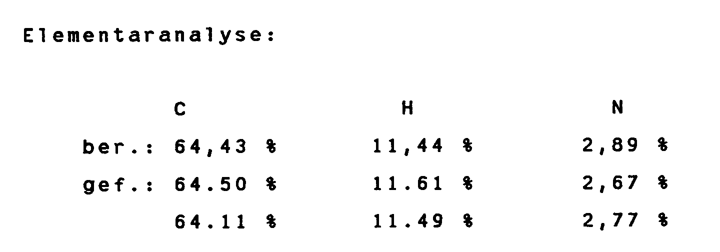

Dünnschichtchromatogramm: (Chloroform/Methanol/1 M Natriumacetat in 25 % Ammoniak 70:40:10)
R_{f} = 0,47

### Beispiel 9: Hexadecyl-(1,1-dimethylpiperidinio-3-yl)methyl-phosphat

### C₂₄H₅₀NO₄P (447.643) * 1 H₂O

Herstellung analog Beispiel 5 aus 10,3 ml (0,11 mol) Phosphoroxychlorid, 24,2 g (0,10 mol) Hexadecanol, 32 + 40 ml Pyridin und 41,0g (0,13 mol) 3-Hydroxymethyl-1,1-dimethylpiperidiniumtosylat.
Ausbeute: 17,2 g (39 %)

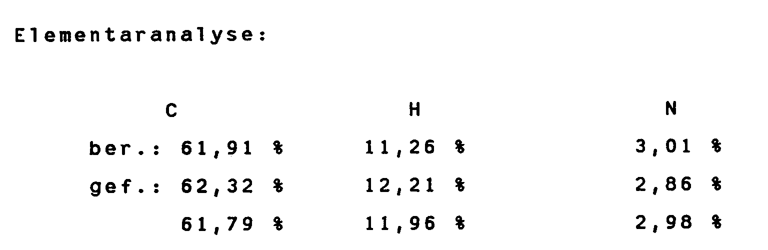

Dünnschichtchromatogramm: (Chloroform/Methanol/1 M Natriumacetat in 25 % Ammoniak 70:40:10)
R_{f} = 0,29

### Beispiel 10: Octadecyl-(1,1-dimethylpiperidinio-3-yl)methyl-phosphat

### C₂₆H₅₄NO₄P (475.697) * H₂O

Herstellung analog Beispiel 5 aus 10,3 ml (0,11 mol) Phosphoroxychlorid,
27,0 g (0,10 mol) Octadecanol, 32 + 40 ml Pyridin und 41,0 g (0,13 mol)
3-Hydroxymethyl-1,1-dimethylpiperidiniumtosylat.
Ausbeute: 16,7 g (35 %)

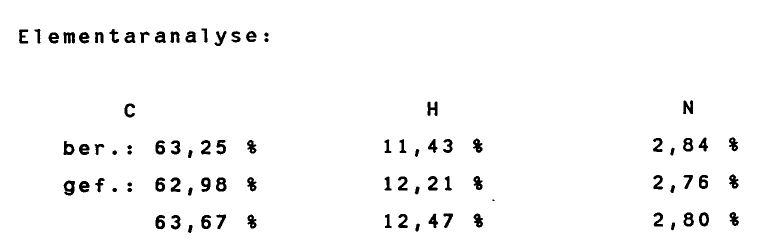

Dünnschichtchromatogramm: (Chloroform/Methanol/1 M Natriumacetat in 25 % Ammoniak 70:40:10)
R_{f} = 0,30

### Beispiel 11: Tetradecyl-(1,1-dimethylhexahydroazepinio-4-yl)-phosphat

### C₂₂H₄₆NO₄P (419,54) * H₂O

Herstellung analog Beispiel 5 aus 9,6 g (45 mmol) Tetradecanol, 4,6 ml (50 mmol) Phosphoroxychlorid, 10 + 20 ml Pyridin und 21,3 g (67,5 mmol) Hydroxy-1,1-dimethylhexahydroazepiniumtosylat. Reinigung durch Flash-Chromatographie an Kieselgel mit Methylenchlorid/Methanol/25 % Ammoniak 70:40:10.
Ausbeute: 2,70 g (15 %)

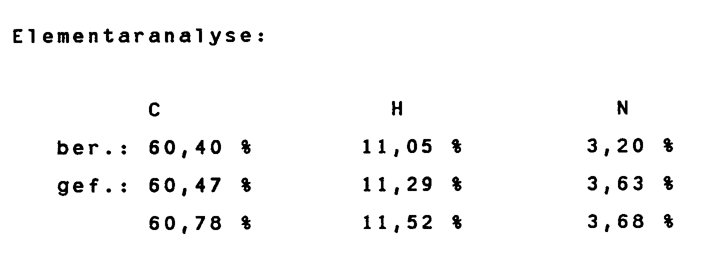

Dünnschichtchromatogramm: (Chloroform/Methanol/1M Natriumacetat in 25 % Ammoniak 70:40:10)
R_{f} = 0,30
(1-Butanol/Eisessig/Wasser 40:10:10)
R_{f} = 0,08

### Beispiel 12: Hexadecyl-(1,1-dimethylhexahydroazepinio-4-yl)-phosphat

### C₂₄H₅₀NO₄P (447,64)

Herstellung analog Beispiel 5 aus 10,8 g (45 mmol) Hexadecanol, 4,6 ml (50 mmol) Phosphoroxychlorid, 10 + 20 ml Pyridin und 21,3 g (67,5 mmol) 4-Hydroxy-1,1-dimethylhexahydroazepiniumtosylat. Reinigung durch Flash-Chromatographie an Kieselgel mit Methylenchlorid/Methanol/25 % Ammoniak 70:30:10.
Ausbeute: 5,0 g (25 %)

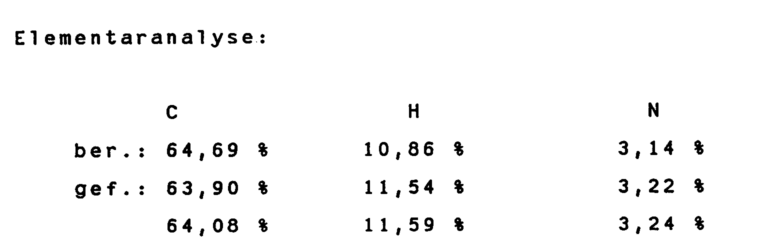

Dünnschichtchromatogramm: (Chloroform/Methanol/25 % Ammoniak 80:25:5)
R_{f} = 0,10
(1-Butanol/Eisessig/Wasser 40:10:10)
R_{f} = 0,10
Schmelzpunkt: >250°C (Zersetzung)

### Beispiel 13: Octadecyl-(1,1-dimethylhexahydroazepinio-4-yl)-phosphat

### C₂₆H₅₄NO₄P (475,695) * 1/2 H₂O

Herstellung analog Beispiel 5 aus 12,1 g (45 mmol) Octadecanol, 4,6 ml (50 mmol Phosphoroxychlorid, 10 + 20 ml Pyridin und 21,3 g (67,5 mmol) 4-Hydroxy-1,1-dimethylhexahydroazepiniumtosylat. Reinigung durch Flash-Chromatographie an Kieselgel mit Methylenchlorid/Methanol/25 % Ammoniak 70:30:10.
Ausbeute: 5,5 g (26 %)

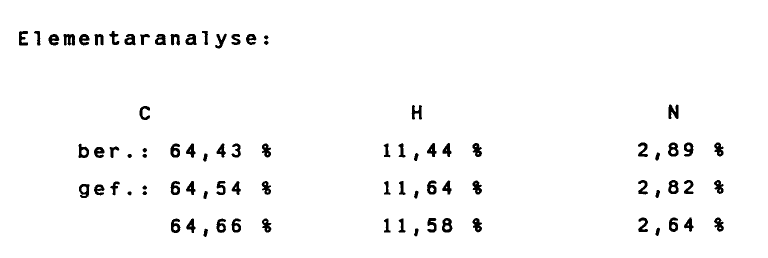

Dünnschichtchromatogramm: (Chloroform/Methanol/1 M Natriumacetat in 25 % Ammoniak 70:40:10)
R_{f} = 0,22
Schmelzpunkt: >250°C (Zersetzung)

### Beispiel 14: cis-Δ⁹-Octadecenyl-(1,1-dimethylhexahydroazepinio-4-yl)-phosphat

### C₂₆H₅₂NO₄P (473,679) * H₂O

Herstellung analog Beispiel 5 aus 12,1 g (45 mmol) cis-Δ⁹-Octadecenol 4,6 ml (50 mmol) Phosphoroxychlorid, 10 + 20 ml Pyridin und 21,3 g 67,5 mmol) 4-Hydroxy-1,1-dimethylhexahydroazepiniumtosylat. Reinigung durch Flash-Chromatographie an Kieselgel mit Methylenchlorid/Methanol/25 % Ammoniak 70:30:10.
Ausbeute: 4,5 g (21 %)

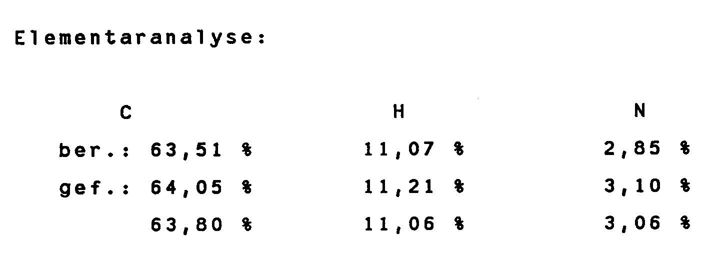

Dünnschichtchromatogramm: (Chloroform/Methanol/25 % Ammoniak 70:40:10)
R_{f} = 0,28
(1-Butanol/Eisessig/Wasser 40:10:10)
R_{f} = 0,10

### Beispiel 15: Eicosyl-(1,1-dimethylhexahydroazepinio-4-yl)-phosphat

### C₂₈H₅₈NO₄P (503,754) * H₂O

Herstellung analog Beispiel 5 aus 13,4 g (45 mmol) Eicosanol, 4,6 ml (50 mmol) Phosphoroxychlorid, 10 + 20 ml Pyridin und 21,3 g (67,5 mmol) 4-Hydroxy-1,1-dimethylhexahydroazepiniumtosylat. Reinigung durch Flash-Chromatographie an Kieselgel mit Methylenchlorid/Methanol/25 % Ammoniak 70:30:10.
Ausbeute: 5,7 g (25 %)

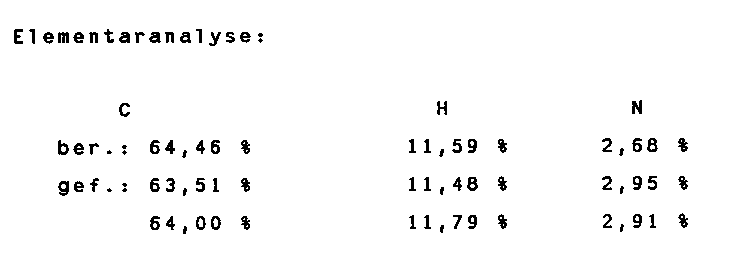

Dünnschichtchromatogramm: (Chloroform/Methanol/25 % Ammoniak 70:40:10)
R_{f} = 0,12

### Beispiel 16: Erucyl-(1,1-dimethylhexahydroazepinio-4-yl)-phosphat

### C₃₀H₆₀NO₄P (529,789) * H₂O

Herstellung analog Beispiel 5 aus 16,2 g (50 mmol) Erucylalkohol, 5,1 ml (55 mmol) Phosphoroxychlorid, 18 + 30 ml Pyridin und 20,5 g (65 mmol) 4-Hydroxy-1,1-dimethylhexahydroazepiniumtosylat. Reinigung durch Flash-Chromatographie an Kieselgel mit Methylenchlorid/Methanol/25% Ammoniak 70:30:10.
Ausbeute: 4,1 g (15 %)

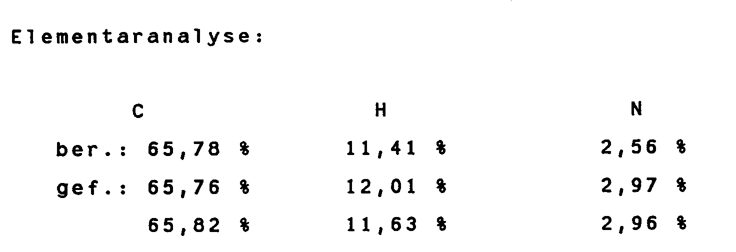

Dünnschichtchromatogramm: (Chloroform/Methanol/1 M Natriumacetat in 25 % Ammoniak 70:40:10)
R_{f} = 0,30

### Beispiel 17: Octadecyl-(1,1-dimethylpyrrolidinio-3-yl)-phosphat

### C₂₄H₅₀NO₄P (447,643) * 1/2 H₂O

Herstellung analog Beispiel 5 aus 3,25 g (12 mmol) Octadecanol, 1,21 ml 13 mmol) Phosphoroxychlorid, 3,7 + 4,8 ml Pyridin und 4,31 g (15 mmol) Hydroxy-1,1-dimethylpyrrolidiniumtosylat. Reinigung des Rohproduktes durch Lösen in 96 % Ethanol und Behandeln mit Ionenaustauscher Amberlite MB 3.
Ausbeute: 1,31 g (25 %)

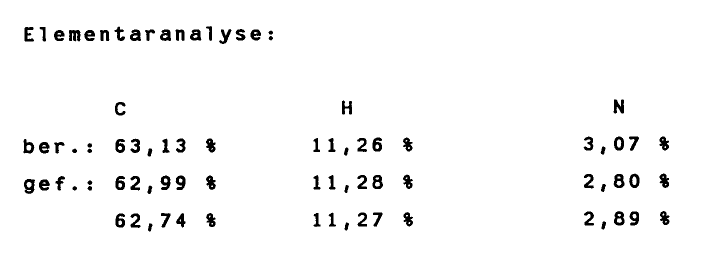

Dünnschichtchromatogramm: (Chloroform/Methanol/1 M Natriumacetat in 25 % Ammoniak 70:40:10)
R_{f} = 0,25

### Beispiel 18: Hexadecyl-2-(1,1-dimethylpyrrolidinio-2-yl)ethyl-phosphat

### C₂₄H₅₀NO₄P (447,643) * H₂O

Herstellung analog Beispiel 5 aus 9,21 g (38 mmol) Hexadecanol, 3,9 ml (42 mmol) Phosphoroxychlorid, 13 + 16 ml Pyridin und 15,8 g (50 mmol) 2-(2-Hydroxyethyl)-1,1-dimethylpyrrolidiniumtosylat. Reinigung durch Lösen in 96 % Ethanol und Behandeln mit Ionenaustauscher Amberlite MB 3.
Ausbeute: 6,0 g (35 %)

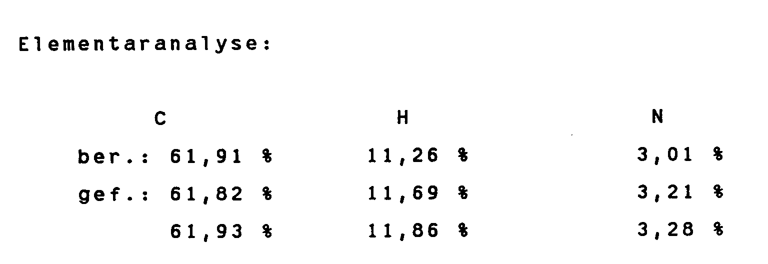

Dünnschichtchromatogramm: (Chloroform/Methanol/1 M Natriumacetat in 25 % Ammoniak 70:40:10)
R_{f} = 0,38

### Beispiel 19: Octadecyl-2-(1,1-dimethylpyrrolidinio-2-yl)ethyl-phosphat

### C₂₆H₅₄NO₄P (475,697) * 1/2 H₂O

Herstellung analog Beispiel 5 aus 10,3 g (38 mmol) Octadecanol, 3,9 ml (42 mmol) Phosphoroxychlorid, 13 + 16 ml Pyridin und 15,8 g (50 mmol) 2-(2-Hydroxyethyl)-1,1-dimethylpyrrolidiniumtosylat. Reinigung durch Lösen in 96 % Ethanol und Behandeln mit Ionenaustauscher Amberlite MB 3.
Ausbeute: 7,8 g (43 %)

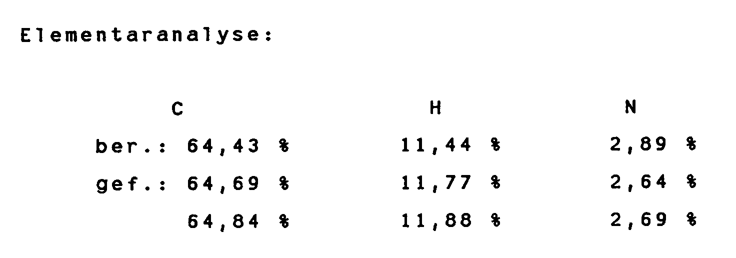

Dünnschichtchromatogramm: (Chloroform/Methanol/1 M Natriumacetat in 25 % Ammoniak 70:40:10)
R_{f} = 0,35

### Beispiel 20: Hexadecyl-(1,1-dimethylpyrrolidinio-2-yl)methyl-phosphat

### C₂₃H₄₈NO₄P (433,616)* 1/2 H₂O

Herstellung analog Beispiel 5 aus 9,21 g (38 mmol) Hexadecanol, 3,9 ml (42 mmol Phosphoroxychlorid, 13 + 16 ml Pyridin und 15,1 g (50 mmol) 2-Hydroxymethyl-1,1-dimethylpyrrolidiniumtosylat. Reinigung durch Lösen in 96 % Ethanol und Behandeln mit Ionenaustauscher Amberlite MB 3.
Ausbeute: 8,3 g (51 %)

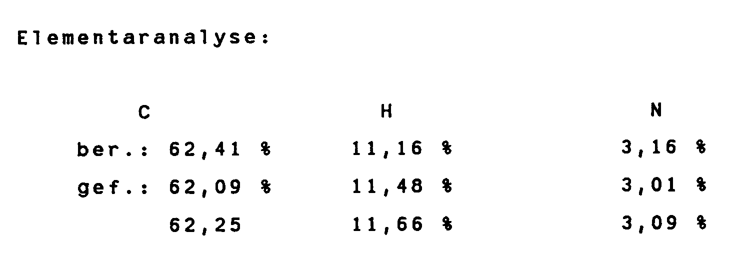

Dünnschichtchromatogramm: (Chloroform/Methanol/1 M Natriumacetat in 25 % Ammoniak 70:40:10)
R_{f} = 0,33

### Beispiel 21: Octadecyl-(1,1-dimethylpyrrolidinio-2-yl)methyl-phosphat

### C₂₅H₅₂NO₄P (461,67) * 1/2 H₂O

Herstellung analog Beispiel 5 aus 10,3 g (38 mmol) Octadecanol, 3,9 ml (42 mmol) Phosphoroxychlorid, 13 + 16 ml Pyridin und 15,1 g (50 mmol) 2-Hydroxymethyl-1,1-dimethylpyrrolidiniumtosylat. Reinigung durch Lösen in 96 % Ethanol und Behandeln mit Ionenaustauscher Amberlite MB 3.
Ausbeute: 9,0 g (52 %)

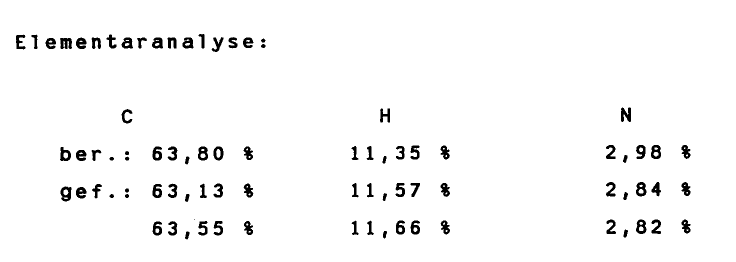

Dünnschichtchromatogramm: (Chloroform/Methanol/1 M Natriumacetat in 25 % Ammoniak 70:40:10)
R_{f} = 0,35

## Patentansprüche

1. Verbindungen der allgemeinen Formel I 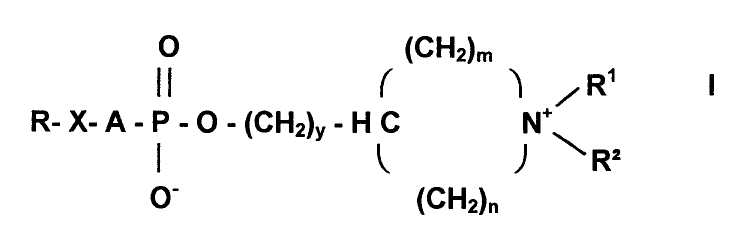 in der R einen geradkettigen oder verzweigten Alkylrest mit 10 bis 24 Kohlenstoffatomen, der auch ein bis drei Doppel- oder Dreifachbindungen enthalten kann,
R¹ und R² unabhängig voneinander Wasserstoff oder je einen geradkettigen, verzweigten cyclischen gesättigten oder ungesättigten Alkylrest mit 1 bis 6 Kohlenstoffatomen, der auch eine Cl-, OH- oder NH₂-Gruppe enthalten kann und wobei zwei dieser Reste auch zu einem Ring verbunden sein können,
A eine Einfachbindung oder eine der Gruppen mit den Formeln 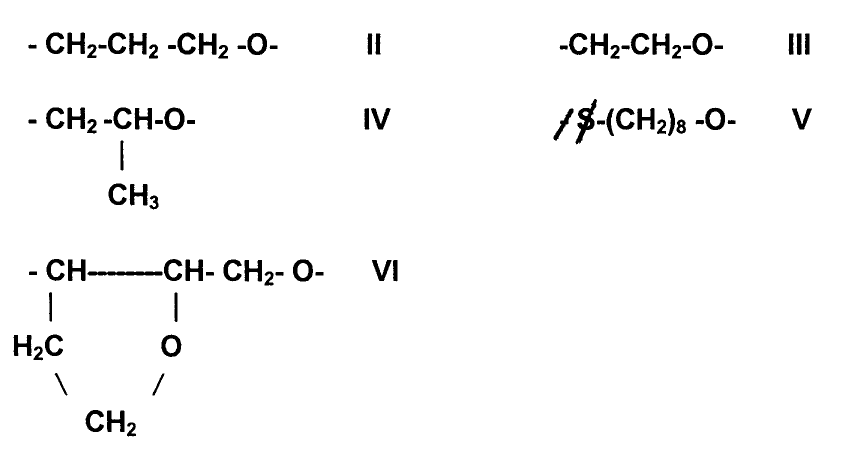 wobei die Gruppen (II) bis (VI) so orientiert sind, daß das Sauerstoffatom an das Phosphoratom der Verbindung (I) gebunden ist, und X ein Sauerstoff- oder Schwefelatom oder NH ist, wenn A eine Einfachbindung ist oder ein Sauerstoff- oder Schwefelatom bedeutet, wenn A eine der Gruppen (II) bis (VI) ist, bedeuten;
y gleich 0 oder eine natürliche Zahl zwischen 1 und 3 ist, m und n unabhängig voneinander 0 oder natürliche Zahlen unter der Bedingung m + n = 2 bis 8 sind.

2. Verbindungen nach Anspruch 1, dadurch gekennzeichnet, daß X ein Sauerstoffatom bedeutet.

3. Verbindungen nach Anspruch 1 oder 2, dadurch gekennzeichnet, daß A eine Einfachbindung bedeutet.

4. Verbindungen nach den Ansprüchen 1 bis 3, dadurch gekennzeichnet, daß R¹ und R² jeweils Methylgruppen sind.

5. Octadecyl-(1,1-dimethylpiperidinio-4-yl)-phosphat.

6. Octadecyl-(1,1-dimethylhexahydroazepinio-4-yl)-phosphat.

7. Octadecyl-(1,1-dimethylpiperidinio-2-yl)methyl-phosphat

8. Erucyl-(1,1-dimethylpiperidinio-4-yl)-phosphat.

9. Erucyl-(1,1-dimethylhexahydroazepinio-4-yl)-phosphat

10. Hexadecyl-(1,1-dimethylpyrrolidinio-2-yl)methyl-phosphat

11. Octadecyl-(1,1-dimethylpyrrolidinio-2-yl)methyl-phosphat

12. Verfahren zur Herstellung einer Verbindung nach einem der Ansprüche 1 bis 11, dadurch gekennzeichnet, daß man eine Verbindung der allgemeinen Formel
R - X - A - H (VII)
in der R, X und A die in Anspruch 1 angegebenen Bedeutungen haben, mit Phosphoroxytrichlorid in Gegenwart einer geeigneten Hilfsbase zur Reaktion bringt und anschließend mit einer Verbindung der allgemeinen Formel 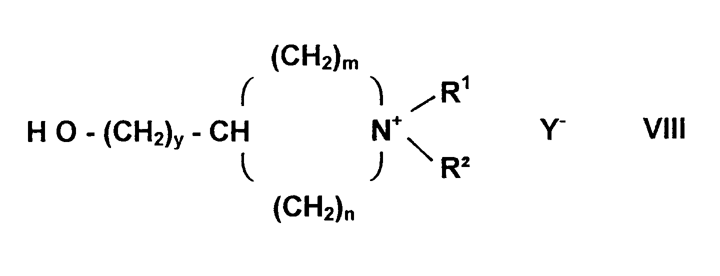 in der R¹ und R², y, m und n die in Anspruch 1 angegebenen Bedeutungen haben und y⁻ Halogenid, Mesylat oder Tosylat bedeutet, zu Verbindungen der allgeminen Formel I umsetzt, gegebenenfalls können Verbindungen der allgemeinen Formel 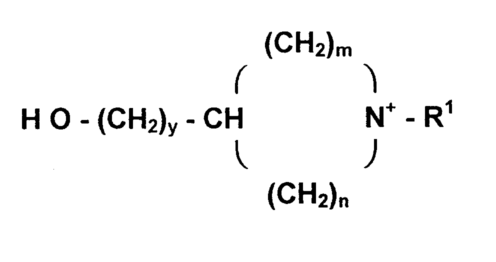 in der R¹, y, m und n die in Anspruch 1 angegebenen Bedeutungen haben, umgesetzt werden, wobei Verbindungen der allgemeinen Formel (I), bei denen R¹ und/oder R² Wasserstoff ist, mit Alkylierungsmitteln R²-Y, bei denen R² die in Anspruch 1 angegebene Bedeutung hat und Y gleich Cl, Brom, Jod, Tosyl oder Mesyl ist, in an sich bekannter Weise umgesetzt werden.

13. Reinigungsverfahren für die Verbindungen der Formel I, dadurch gekennzeichnet, daß man eine Lösung der Verbindungen gemäß Formel I, die mittels bekannter Verfahren oder gemäß einem Verfahren gemäß Anspruch 12 hergestellt wurden, in einem organischen Lösungsmittel mit einem Mischbettionenaustauscher, oder nacheinander oder gleichzeitig mit einem sauren oder basischen Ionenaustauscher behandelt.

14. Arzneimittel, dadurch gekennzeichnet, daß es als Wirkstoff eine oder mehrere Verbindungen nach einem der Ansprüche 1 bis 11 oder ein pharmazeutisch verträgliches Salz hiervon, gegebenenfalls zusammen mit pharmazeutisch üblichen Träger-, Hilfs-, Füll- und Verdünnungsmitteln enthält, wobei die Menge an Wirkstoff zwischen 50 mg und 250 mg liegt.

15. Arzneimittel , dadurch gekennzeichnet, daß es als Wirkstoff
Octadecyl-(1,1-dimethylpiperidinio-4-yl)-phosphat,
Octadecyl-(1,1-dimethylhexahydroazepinio-4-yl)-phosphat,
Octadecyl-(1,1-dimethylpiperidinio-2-yl)methyl-phosphat,
Erucyl-(1,1-dimethylpiperidinio-4-yl)-phosphat,
Erucyl-(1,1-dimethylhexahydroazepinio-4-yl)-phosphat,
Hexadecyl-(1,1-dimethylpyrrolidinio-2-yl)methyl-phosphat,
Octadecyl-(1,1-dimethypyrrolidinio-2-yl)methyl-phosphat
enthält, wobei die Menge des Wirkstoffs jeweils zwischen 50 mg und 250 mg liegt.

16. Arzneimittel nach Anspruch 5 und 15, dadurch gekennzeichnet, daß es als Wirkstoff Octadecyl-(1,1-dimethylpiperidinio-4-yl)phosphat enthält, wobei die Menge des Wirkstoffs zwischen 50 mg und 250 mg liegt.

17. Verwendung von Verbindungen gemäß der Ansprüche 1 bis 11 zur Herstellung von Arzneimitteln zur Bekämpfung von Tumoren.

18. Verwendung von Verbindungen gemäß der Ansprüche 1 bis 11 zur Herstellung von Arzneimitteln zur Bekämpfung von Protozoen- und von Pilzerkrankungen, insbesondere der Leishmaniasis.

19. Verwendung von Verbindungen gemäß der Ansprüche 1 bis 11 zur Herstellung von Arzneimitteln zur Therapie von Autoimmunkrankheiten, insbesondere von multipler Sklerose.

20. Verwendung von Verbindungen gemäß der Ansprüche 1 bis 11 zur Herstellung von Arzneimitteln zur Therapie von Hauterkrankungen, insbesondere von Psoriasis.

21. Verwendung von Verbindungen gemäß der Ansprüche 1 bis 11 zur Herstellung von Arzneimitteln zur Therapie von Knochenmarksschädigungen durch Behandlung mit Zytostatika und anderen knochenmarksschädigenden Wirkstoffen.

22. Verfahren zur Herstellung eines Antitumormittels, dadurch gekennzeichnet, daß man eine Formulierung herstellt, die als Wirkstoff eine oder mehrere Verbindungen nach einem der Ansprüche 1 bis 11 enthält, gegebenenfalls zusammen mit pharmazeutisch üblichen Träger-, Hilfs-, Füll- und Verdünnungsmitteln.

## Claims

1. Compounds of the general formula I 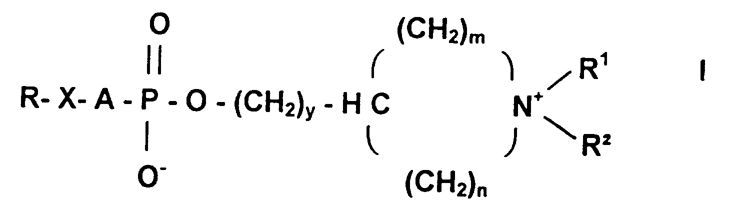 in which R is a straight-chain or branched alkyl radical having 10 to 24 carbon atoms, which can also contain one to three double or triple bonds,
R¹ and R² independently of one another are hydrogen or each is a straight-chain, branched, cyclic, saturated or unsaturated alkyl radical having 1 to 6 carbon atoms, which can also contain a Cl, OH or NH₂ group and where two of these radicals can also be bonded to give a ring,
A is a single bond or one of the groups having the formulae 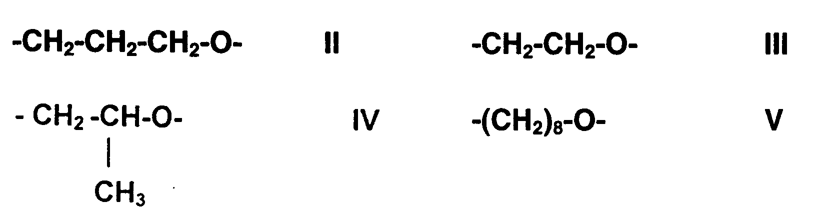 where the groups (II) to (VI) are arranged such that the oxygen atom is bonded to the phosphorus atom of the compound (I), and X is an oxygen or sulphur atom or NH if A is a single bond or is an oxygen or sulphur atom if A is one of the groups (II) to (VI);
y is 0 or a natural number between 1 and 3, m+n independently of one another are 0 or natural numbers with the condition m+n = 2 to 8.

2. Compounds according to Claim 1, characterized in that X is an oxygen atom.

3. Compounds according to Claim 1 or 2, characterized in that A is a single bond.

4. Compounds according to Claims 1 to 3, characterized in that R¹ and R² are each methyl groups.

5. Octadecyl 1,1-dimethylpiperidinio-4-yl phosphate.

6. Octadecyl 1,1-dimethylhexahydroazepinio-4-yl phosphate.

7. Octadecyl (1,1-dimethylpiperidinio-2-yl)methyl phosphate.

8. Erucyl 1,1-dimethylpiperidinio-4-yl phosphate.

9. Erucyl 1,1-dimethylhexahydroazepinio-4-yl phosphate.

10. Hexadecyl (1,1-dimethylpyrrolidinio-2-yl)methyl phosphate.

11. Octadecyl (1,1-dimethylpyrrolidinio-2-yl)methyl phosphate.

12. Process for the preparation of a compound according to one of Claims 1 to 11, characterized in that a compound of the general formula
R - X - A - H (VII)
in which R, X and A have the meanings indicated in Claim 1, is reacted with phosphorus oxychloride in the presence of a suitable auxiliary base and then reacted with a compound of the general formula 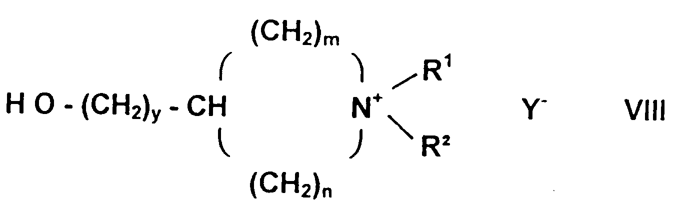 in which R¹ and R², y, m and n have the meanings indicated in Claim 1 and Y⁻ is halide, mesylate or tosylate, to give compounds of the general formula I, optionally compounds of the general formula 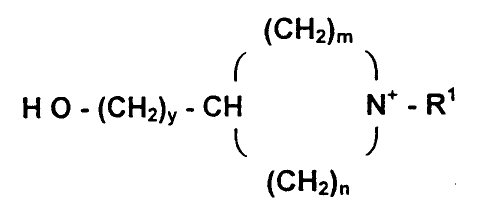 in which R¹, y, m and n have the meanings indicated in Claim 1, can be reacted, compounds of the general formula (I) in which R¹ and/or R² is hydrogen being reacted in a manner known per se with alkylating agents R²-Y, in which R² has the meaning indicated in Claim 1 and Y is Cl, bromine, iodine, tosyl or mesyl.

13. Purification process for the compounds of the formula I, characterized in that a solution of the compounds according to formula I which has been prepared by means of known processes or by a process according to Claim 12 is treated in an organic solvent with a mixed bed ion exchanger, or successively or simultaneously with an acidic or basic ion exchanger.

14. Medicament, characterized in that, as active compound, contains one or more compounds according to one of Claims 1 to 11 or a pharmaceutically tolerable salt thereof, if appropriate together with pharmaceutically customary excipients, auxiliaries, fillers and diluents, the amount of active compound being between 50 mg and 250 mg.

15. Medicament, characterized in that, as active compound, contains
octadecyl 1,1-dimethylpiperidinio-4-yl phosphate,
octadecyl 1,1-dimethylhexahydroazepinio-4-yl phosphate,
octadecyl (1,1-dimethylpiperidinio-2-yl)methyl phosphate,
erucyl 1,1-dimethylpiperidinio-4-yl phosphate,
erucyl 1,1-dimethylhexahydroazepinio-4-yl phosphate,
hexadecyl (1,1-dimethylpyrrolidinio-2-yl)methyl phosphate or
octadecyl (1,1-dimethylpyrrolidinio-2-yl)methyl phosphate.
the amount of active compound in each case being between 50 mg and 250 mg.

16. Medicament according to Claims 5 and 15, characterized in that, as active compound, it contains octadecyl 1,1-dimethylpiperidinio-4-yl phosphate, the amount of active compound being between 50 mg and 250 mg.

17. Use of compounds according to Claims 1 to 11 for the production of medicaments for the control of tumours.

18. Use of compounds according to Claims 1 to 11 for the production of medicaments for the control of protozoal and of fungal disorders, in particular of leishmaniasis.

19. Use of compounds according to Claim 1 to 11 for the production of medicaments for the therapy of autoimmune diseases, in particular of multiple sclerosis.

20. Use of compounds according to Claims 1 to 11 for the production of medicaments for the therapy of skin disorders, in particular of psoriasis.

21. Use of compounds according to Claims 1 to 11 for the production of medicaments for the therapy of bone marrow damage as a result of treatment with cytostatics and other active compounds which damage the bone marrow.

22. Process for the production of an anti-tumour composition characterized in that a formulation is prepared which, as active compound, contains one or more compounds according to one of Claims 1 to 11, if appropriate together with pharmaceutically customary excipients, auxiliaries, fillers and diluents.

## Revendications

1. Composés de formule générale I 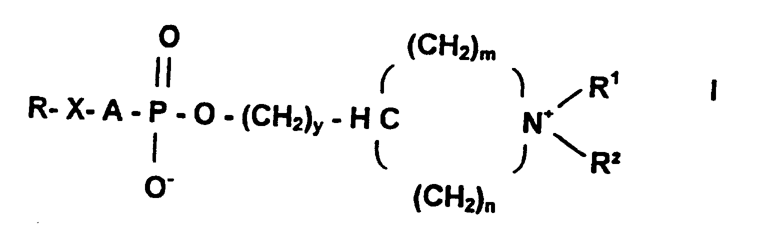 dans laquelle R représente un radical alkyle linéaire ou ramifié ayant de 10 à 24 atomes de carbone, qui peut également contenir une à trois doubles ou triples liaisons,
R¹ et R² représentent, indépendamment l'un de l'autre, un hydrogène ou chacun un radical alkyle, linéaire, ramifié ou cyclique, saturé ou insaturé, ayant de 1 à 6 atomes de carbone, qui peut également contenir un groupe Cl, OH ou NH₂ et où deux de ces radicaux peuvent également être reliés à un cycle,
A représente une liaison simple ou l'un des groupes de formules 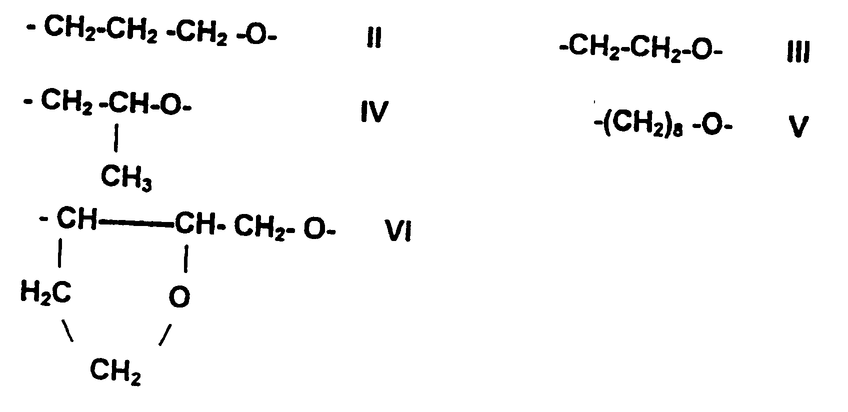 les groupes (II) à (VI) étant orientés de telle sorte que l'atome d'oxygène soit lié à l'atome de phosphore du composé (I), et X est un atome d'oxygène ou de souffre ou NH, si A est une liaison simple ou représente un atome d'oxygène ou de souffre, si A est l'un des groupes (II) à (VI);
y vaut 0 ou est un entier naturel compris entre 1 et 3, m et n, indépendamment l'un de l'autre, valent 0 ou sont des entiers naturels répondant à la condition m + n = 2 à 8.

2. Composés selon la revendication 1, caractérisés en ce que X représente un atome d'oxygène.

3. Composés selon la revendication 1 ou 2, caractérisés en ce que A représente une liaison simple.

4. Composés selon les revendications 1 à 3, caractérisés en ce que R¹ et R² sont, dans chaque cas, des groupes méthyle.

5. Octadécyl-(1,1-diméthylpipéridinio-4-yl)-phosphate.

6. Octadécyl-(1,1-diméthylhexahydroazépinio-4-yl)-phosphate.

7. Octadécyl-(1,1-diméthylpipéridinio-2-yl)méthyl-phosphate.

8. Erucyl-(1,1-diméthylpipéridinio-4-yl)-phosphate.

9. Erucyl-(1,1-diméthylhexahydroazépinio-4-yl)-phosphate.

10. Hexadécyl-(1,1-diméthylpyrrolidinio-2-yl)méthyl-phosphate.

11. Octadécyl-(1,1-diméthylpyrrolidinio-2-yl)méthyl-phosphate.

12. Procédé de préparation d'un composé selon l'une quelconque des revendications 1 à 11, caractérisé en ce que l'on fait réagir un composé de formule générale
R-X-A-H (VII)
dans laquelle R, X et A ont les significations indiquées à la revendication 1, avec de l'oxychlorure de phosphore en présence d'une base auxiliaire appropriée, et on met ensuite à réagir avec un composé de formule générale 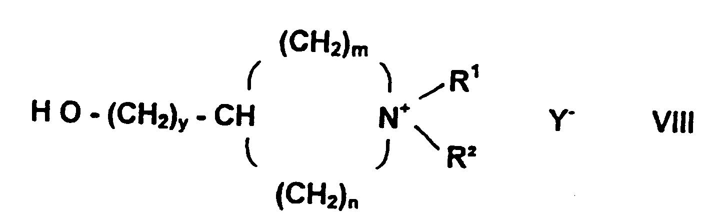 dans laquelle R¹ et R², y, m et n ont les significations indiquées à la revendication 1, et Y⁻ représente un halogénure, un mésylate ou un tosylate, pour donner des composés de formule générale I, en faisant éventuellement réagir des composés de formule générale 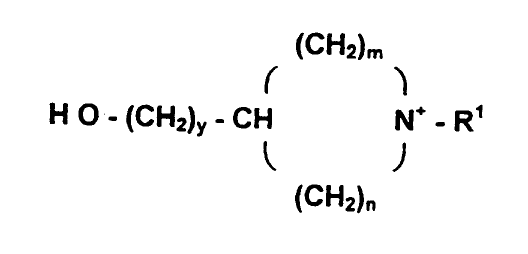 dans laquelle R¹, y, m et n ont les significations indiquées à la revendication 1, des composés de formule générale (I), dans lesquels R¹ et/ou R² sont un hydrogène, étant mis à réagir avec des agents d'alkylation R²-Y, dans lesquels R² a la signification indiquée à la revendication 1 et Y est Cl, un brome, un iode, un tosyle ou un mésyle.

13. Procédé de purification pour les composés de formule I, caractérisé en ce qu'une solution des composés selon la formule I, qui ont été préparés par un procédé connu ou d'après un procédé selon la revendication 12 est traitée, dans un solvant organique, par un échangeur d'ions en lit mixte, ou successivement ou simultanément par un échangeur d'ions acide ou basique.

14. Médicament, caractérisé en ce qu'il contient, en tant que substance active, un ou plusieurs composés selon l'une quelconque des revendications 1 à 11 ou un sel pharmaceutiquement acceptable de ceux-ci, éventuellement conjointement avec des supports, des auxiliaires, des charges et des diluants pharmaceutiquement usuels, la quantité de substance active étant comprise entre 50 mg et 250 mg.

15. Médicament, caractérisé en ce qu'il contient, en tant que substance active,
l'octadécyl-(1,1-diméthylpipéridinio-4-yl)-phosphate,
l'octadécyl-(1,1-diméthylhexahydroazépinio-4-yl)-phosphate,
l'octadécyl-(1,1-diméthylpipéridinio-2-yl)méthyl-phosphate,
l'érucyl-(1,1-diméthylpipéridinio-4-yl)-phosphate,
l'érucyl-(1,1-diméthylhexahydroazépinio-4-yl)-phosphate,
l'hexadécyl-(1,1-diméthylpyrrolidinio-2-yl)méthyl-phosphate,
l'octadécyl-(1,1-diméthylpyrrolidinio-2-yl)méthyl-phosphate
la quantité de la substance active étant à chaque fois comprise entre 50 mg et 250 mg.

16. Médicament selon les revendications 5 et 15, caractérisé en ce qu'il contient, en tant que substance active, l'octadécyl-(1,1-diméthylpipéridinio-4-yl)phosphate, la quantité de la substance active étant comprise entre 50 mg et 250 mg.

17. Utilisation de composés selon les revendications 1 à 11 pour la préparation de médicaments destinés à la lutte contre les tumeurs.

18. Utilisation de composés selon les revendications 1 à 11 pour la préparation de médicaments destinés à la lutte contre des maladies liées à des protozoaires et à des champignons, en particulier la leishmaniose.

19. Utilisation de composés selon les revendications 1 à 11 pour la préparation de médicaments destinés à la thérapie de maladies auto-immunes, en particulier de la sclérose en plaques.

20. Utilisation de composés selon les revendications 1 à 11 pour la préparation de médicaments destinés à la thérapie de dermatoses, en particulier du psoriasis.

21. Utilisation de composés selon les revendications 1 à 11 pour la préparation de médicaments destinés à la thérapie de lésions de la moelle osseuse par traitement avec des cytostatiques et d'autres substances affectant la moelle osseuse.

22. Procédé de préparation d'un agent antitumoral, caractérisé en ce que l'on prépare une formulation qui contient, en tant que substance active, un ou plusieurs composés selon l'une quelconque des revendications 1 à 11, éventuellement conjointement avec des supports, des auxiliaires, des charges et des diluants pharmaceutiquement usuels.
